# EUROPEAN PATENT APPLICATION

(11) **EP 4 521 114 A1**
(43) Date of publication of application: **12.03.2025**
(21) Application number: 23205863.6
(22) Date of filing: 25.10.2023
(51) Int. Cl.: G01N 33/53, C12Q 1/6804, C12Q 1/6823

(54) **AFFINITY REAGENT, MARKER AND METHOD FOR ANALYSING A BIOLOGICAL SAMPLE**

(30) Priority: 06.09.2023 EP 23195848
(71) Applicant: Leica Microsystems CMS GmbH, 35578 Wetzlar (DE)
(72) Inventor: Alsheimer, Soeren, 35578 Wetzlar (DE)
(74) Representative: Schaumburg und Partner Patentanwälte mbB

(57) **Abstract**

An affinity reagent (102) for analysing a biological sample is provided. The affinity reagent (102) comprises a backbone (106) and a barcode oligonucleotide (108) attached to the backbone (106). The backbone (106) consists essentially of a nucleic acid analogue. The backbone (106) is configured to specifically bind to the target analyte (114) by its complex structure. The barcode oligonucleotide (108) consists essentially of a nucleic acid analogue. In a further aspect a marker (100, 206) comprising the affinity reagent (102) and a label (104, 202) is provided. Further, a method for analysing a biological sample with the marker (100, 206) is provided.

## Description

### Technical field

The invention relates to an affinity reagent for analysing a biological sample comprising a backbone and a barcode oligonucleotide. In further aspects, a marker comprising the affinity reagent and a method for analysing a biological sample with the marker are provided.

### Background

Affinity reagents comprise a chemically heterogeneous group and include for example antibodies, nanobodies, nucleic acid probes, and aptamers. Affinity reagents are commonly used in life science research and diagnostic applications to detect target molecules also referred to as analytes. To this end a variety of detectable labels is used, which comprise at least one labelling moiety.

Aptamers are typically short nucleic acid sequences that fold into 3-dimensional structures, which are able to bind a target analyte with high affinity and specificity. The use of barcoded affinity reagents such as antibodies, nanobodies, aptamers, or *in situ* probes, which are connected to an oligonucleotide sequence that is unique to said affinity reagent and may contain one or more predetermined sequences that can be hybridised by detectable labels that comprise a complementary barcode, is an effective strategy for cyclical labelling. Such cyclical labelling is used when biological samples shall be analysed for the presence of a high number of target analytes.

Fluorescence microscopy allows for imaging a biological sample with high spatial resolution but involves only a low number of different fluorescent dyes, typically between 1 and 5. The available markers comprising the dyes have to include markers that are used to identify cell types, functional markers like protein-of-interest, and general morphological markers in the same experiment. This means that cell types in most imaging experiments are merely poorly identified. This also means that rather broad multi cell type populations are being studied, which severely limits the predictive power and translational value of the results generated. While modern approaches that allow for a much more reliable and robust identification of cell types, e.g. based on the analysis of genetic regulatory networks (GRNs), exist, they require a much higher number of markers with distinguishable dyes to be readout from the sample.

While in the adjacent field of cytometry, mass cytometry and imaging mass cytometry techniques can distinguish between around 12 to 30 different markers, they do so with a low spatial resolution.

Spatial profiling techniques based on RNA capture can distinguish a number of different markers several orders of magnitude higher, albeit at an even lower spatial resolution as they are based on hybridizing oligonucleotides to the sample and then selectively releasing bound oligonucleotides in a region-selective fashion followed by next-generation sequencing of the released oligonucleotides.

### Summary

It is an object to provide a barcoded affinity reagent and a marker that enable efficient repeated staining of a biological sample and a method for analysing a biological sample with the marker.

The aforementioned object is achieved by the subject-matter of the independent claims. Advantageous embodiments are defined in the dependent claims and the following description.

An affinity reagent for analysing a biological sample is provided. The affinity reagent comprises a backbone and a barcode oligonucleotide attached to the backbone. The backbone consists essentially of a nucleic acid analogue. The backbone is configured to specifically bind to the target analyte by its complex structure. The barcode oligonucleotide consists essentially of a nucleic acid analogue.

In particular, the barcode oligonucleotide is configured to hybridise a label oligonucleotide of a detectable label in order to attach the label to the affinity reagent. The complex structure of the backbone of the affinity reagent may be a secondary, tertiary or quaternary structure of the backbone nucleic acid analogue. Thus, the backbone of the affinity reagent has a secondary, tertiary or quaternary structure, in particular. In particular, the affinity reagent binds specifically to the target analyte due to the complex structure of the backbone of the affinity reagent. Specifically, individual, discontinuous nucleotides of the nucleic acid analogue of the backbone may bind to the target analyte, rather than continuous nucleotides of a sequence of the nucleic acid analogue hybridising to the target analyte, in particular a complementary sequence of the target analyte. This enables binding of the affinity reagent with high specificity. In particular, the backbone of the affinity reagent, e.g. an aptamer, may bind to its target by paratope-epitope interactions and not via hybridization of complementary nucleic sequences.

An oligonucleotide in particular refers to a nucleotide sequence of any length as well as to nucleic acid based structures that may be unipartite or multipartite in nature.

Generally, nucleic acid analogues are compounds which are structurally similar to naturally occurring RNA and DNA. Nucleic acids are chains of nucleotides, which are composed of three parts: a phosphate backbone, a pentose sugar, either ribose or deoxyribose, and one of four nucleobases. An analogue may have any of these altered. Thus, a nucleic acid analogue may be a non-naturally occurring, modified, artificial, or xeno nucleic acids (XNA), in particular. Whereas natural nucleic acids or naturally occurring nucleic acids (DNA and RNA) are generally sensitive to natural degradation agents such as nucleases, nucleic acid analogues are generally resistant to these degradation agents such as nucleases. Thus, this enables providing an affinity reagent resistant to degradation by natural degradation agents, such as nucleases, and therefore a particular robust affinity reagent. In particular, this enables repeated staining of biological samples based on the affinity reagent with degradation agents without causing denaturation or degradation of the affinity reagent. The biological sample may be a tissue section, for example. Further examples of the biological sample include a cell, a cell culture, or a sample of a bodily liquid.

The barcode oligonucleotide is in particular covalently attached to the backbone of the affinity reagent. The term backbone of the affinity reagent is not limiting to a particular part or element. For example, the backbone of the affinity reagent does not exclusively refer to a phosphate backbone of the nucleic acid analogue. Thus, the barcode oligonucleotide may be attached to any of a phosphate backbone, a pentose sugar, or a nucleobase, or their respective analogues, of the nucleic acid analogue of the backbone of the affinity reagent.

Preferably, the backbone comprises 10 to 100 nucleotides. This enables particular compact affinity reagents, that are easy and fast to introduce into a biological sample and nevertheless have high specificity to a respective target analyte. In particular, these nucleotides may be nucleotide analogues. Preferably, the backbone comprises 30 to 80 nucleotides. In particular and in a related measure, the backbone may be 10 to 30 kDa in size. In a particular embodiment, the backbone may be multimerised and comprise several copies of the backbone comprising 10 to 100 nucleotides.

Preferably, the nucleic acid analogue of the backbone comprises at least one of the following, a locked nucleic acid (LNA), a morpholino, a nucleic acid backbone comprising phosphorothioate (PT) modifications or may comprise a PT-DNA backbone in its entirety, a peptide nucleic acid (PNA), a bridged nucleic acid (BNA), a 2'O-methyl-stubstituted RNA, a glycol nucleic acid (GNA), a hexitol nucleic acid (HNA), an altritol nucleic acid (ANA), a threose nucleic acid (TNA), a cyclohexene nucleic acids (CeNA), a fluoroarabinonucleic acid (FANA), a L- deoxyribonucleic acid. A L- deoxyribonucleic acid comprises a L-pentose backbone, in particular. This enables a robust backbone of the affinity reagent.

Preferably, the nucleic acid analogue of the barcode oligonucleotide comprises at least one of the following, a locked nucleic acid (LNA), a morpholino, a nucleic acid backbone comprising phosphorothioate (PT) modifications or may comprise a PT-DNA backbone in its entirety, a peptide nucleic acid (PNA), a bridged nucleic acid (BNA), a 2'O-methyl-stubstituted RNA, a glycol nucleic acid (GNA), a hexitol nucleic acid (HNA), an altritol nucleic acid (ANA), a threose nucleic acid (TNA), a cyclohexene nucleic acids (CeNA), a fluoroarabinonucleic acid (FANA), a L- deoxyribonucleic acid. A L-deoxyribonucleic acid comprises a L-pentose backbone, in particular. This enables a robust barcode oligonucleotide of the affinity reagent.

The use of LNA, PT-DNA, morpholino, or PNA for the backbone of the affinity reagent or the barcode oligonucleotides may be particularly preferred as these XNA are not only nuclease resistant, which improves their stability, but also bind to a complementary DNA with higher affinity.

It is particularly preferred that the backbone is an aptamer. This enables efficiently generating backbones of the affinity reagent that are specific to a range of target analytes, such as various proteins. Aptamers may be generated in various ways, with SELEX being the most typical route. Additionally, the nucleic acid analogue backbone of the aptamer may preferably comprise modifications, such as glycosylation or the modification of nucleobases with amino acids or chemical moieties that resemble amino acid side chains. Such modifications have been introduced for example on slow OFF-rate modified aptamers (SOMAmers) or aptabodies, and improve target binding. Specifically, SOMAmers exhibit much slower OFF-rates than most aptamers.

Aptamers are next generation affinity reagents that provide numerous advantages over antibodies including lower production cost, higher batch-to-batch consistency, digital storage, on demand synthesis (i.e. oligonucleotide synthesis), easy functionalization, low immunogenicity, free selection of desired epitope, short development times. Aptamers are generally found by using SELEX. Traditional or classical SELEX has been described in US 5,475,096 and US 5,279,163. Multiple SELEX methods have been developed in the meantime that improve upon certain aspects of the original method and allow the development of aptamers, with improved efficiency, better OFF-rates, higher affinities, or further functionalizations like aptamers that lock-on to their targets covalently (e.g. Chemi-SELEX; Photo-SELEX). Large libraries of aptamers or modified aptamers such as the slow OFF-rate modified aptamers (SOMAmers) are commercially available from SOMALogic (Boulder, USA) or other aptamer companies. In the context of microscopy the small size of aptamers is a dedicated advantage as it allows for much better penetration into thicker specimens. Likewise, in previous applications further advantages of the use of aptamers with respect to microscopic applications or proteomics using a microscopic readout have been disclosed in the EP applications with the respective application numbers 23178065.1 and 23183879.8, the complete respective contents of these documents are incorporated herein by reference.

Preferably, the barcode oligonucleotide is adapted to be connected to a label via a label oligonucleotide, the label comprising the label oligonucleotide and at least one labelling moiety, wherein preferably the backbone and the barcode oligonucleotide are resistant to a degradation agent and the label oligonucleotide is sensitive to the degradation agent. This enables easily adding a label to the affinity reagent in order to generate a marker.

As an example, a labelling moiety may be one of the following: a fluorescent dye, a fluorescent structure, a TauTag as disclosed in the PCT application with the application number PCT/EP2022/060035, the complete content of which is incorporated herein by reference, a combinatorial (fluorescent) label as disclosed in WO 2022/242849 A1 and WO 2022/242887 A1, and in the PCT application with the application number PCT/EP2023/075470, the complete respective contents of these documents are incorporated herein by reference, a responsive label as disclosed in the EP application with the application number 23192184.2, the complete content of which is incorporated herein by reference, an oligonucleotide label configured for sequencing based readout, an affinity tag configured to perform affinity purification (e.g. FLAG-, Strep II-, Myc-tag), a polystyrene bead, a metal tag, an enzyme (e.g. horse radish peroxidase), a radioactive tag, a bioluminescence tag, a FRET tag, a Raman label, a polyyne-based label, an SRS-tag.

In a further aspect a marker for analysing a biological sample is provided. The marker comprises the affinity reagent and a label comprising a label oligonucleotide and at least one labelling moiety. The label oligonucleotide and the barcode oligonucleotide of the affinity reagent are complementary to each other, and the label is hybridised to the affinity reagent based on complementary base pairing between the label oligonucleotide and the barcode oligonucleotide. In particular, a continuous sequence of the label oligonucleotide binds to a complementary continuous sequence of the barcode oligonucleotide. One of the label oligonucleotide and the barcode oligonucleotide is sensitive to a degradation agent and the other one of the label oligonucleotide and the barcode oligonucleotide is resistant to the degradation agent. Thus, the sensitive oligonucleotide is configured to be degraded by the degradation agent. The resistant oligonucleotide is configured to not be degraded by the degradation agent. This enables selectively degrading the label oligonucleotide or the barcode oligonucleotide.

In particular, this enables selectively degrading the sensitive one of the label oligonucleotide and the barcode oligonucleotide and therefore removing the label from the affinity reagent, in particular from the affinity reagent backbone, by addition of the degradation agent. Preferably, the affinity reagent, in particular the affinity reagent backbone, remains intact in the presence of the degradation agent. In particular, the affinity reagent backbone may continue to be configured to specifically bind to the target analyte in the presence of the degradation agent. Thus, preferably, the affinity reagent backbone is resistant to the degradation agent.

The degradation agent causes degradation only of the sensitive one of the barcode oligonucleotide and the label oligonucleotide, in particular, the degradation agent may digest or fragment the sensitive oligonucleotide. This degradation generally results in loss of function of the oligonucleotide, in particular, the sensitive oligonucleotide no longer binds or is no longercapable of bindingto the resistant oligonucleotide. In other words, the sensitive oligonucleotide is configured to be (specifically) degraded by the degradation agent. Preferably, the degradation of the sensitive oligonucleotide is irreversible. Thus, the degradation agent preferably causes the label, in particular the label oligonucleotide, to be removed from the barcode oligonucleotide and render the barcode oligonucleotide accessible to another label in another round of labelling. The specific removal of the label from the affinity reagent whilst leaving the affinity reagent intact enables easy iteration of adding and removing labels to the affinity reagents in cyclical staining methods.

In the sense of this document the virtual assignment or mapping of an affinity reagent to a detectable label or the physical association of both, may represent a marker. A marker in the sense of this document may in particular be used to mark an analyte for image-based readout (e.g. microscopy, plate reading), colorimetric readout, cytometric readout, densitometric readout, or visual inspection. Such markers are used for a variety of applications including but not limited to Western blotting, ELISA assays, ELISpot assays, FluoroSpot assays, LUMINEX^{™} assays, AVEXIS assays, immunofluorescence, immunocytochemistry, immunohistochemistry, proximity ligation assays, proximity extension assays, protein purification, protein quantitation, proteomic analysis, biomarker analysis, pathogen detection and identification, and cytometry. Presently, most markers used in life science research and diagnostics for protein detection are based on antibodies.

The sequence of the barcode oligonucleotide and the label oligonucleotide may preferably be specific to the respective affinity reagent and/or the respective labelling moiety.

Preferably, the label oligonucleotide is sensitive to the degradation agent and the affinity reagent is resistant to the degradation agent. Thus, preferably, the barcode oligonucleotide and the backbone of the affinity reagent are resistant to the degradation agent. This enables a robust affinity reagent particularly suited for cyclical staining methods.

Preferably, the label oligonucleotide consists essentially of a natural nucleic acid. Thus, the label oligonucleotide is preferably not a nucleic acid analogue. This enables a label oligonucleotide that is particularly sensitive to the degradation agent. The natural nucleic acid is generally sensitive to naturally occurring degradation agents.

Preferably, the at least one labelling moiety is optically detectable. This enables easy detection of the marker. For example, the labelling moiety is a fluorophore or a combination of a plurality of fluorophores. The optically detectable labelling moiety may be detected by means of a microscope or any other suitable optical readout.

Alternatively, the labelling moiety may be an alkyne detectable by Raman spectroscopy, for example.

Preferably, the label comprises a plurality of labelling moieties. The plurality of labelling moieties may all have the same detectable properties or they may have different properties. This enables labels that are particularly easy to detect or labels that are particularly easy to distinguish from each other.

Preferably, the degradation agent is a nuclease. For example, the degradation agent may be a DNase I. This enables easy degradation of the sensitive oligonucleotide, in particular, a naturally occurring nucleic acid, whereas the resistant oligonucleotide may be a nucleic acid analogue.

Alternatively, the degradation agent may be iodine, for example. In this case, the sensitive oligonucleotide may be a phosphorothioate modified nucleic acid analogue sensitive to iodine and the resistant oligonucleotide may be a naturally occurring nucleic acid (e.g. D-DNA) or a nucleic acid analogue resistant to iodine such as L-DNA.

Particularly preferred combinations of label oligonucleotides, barcode oligonucleotides, backbones of the affinity reagent and degradation agents are: a backbone of the affinity reagent consisting essentially of a L-deoxyribonucleic acid, a barcode oligonucleotide consisting essentially of a locked nucleic acid, a label oligonucleotide consisting essentially of a D-deoxyribonucleic acid, and a nuclease such as DNase I as degradation agent. In this case, the D-DNA label oligonucleotide is sensitive to the degradation agent and is readily digested by the nuclease upon its addition. The remaining nucleic acids are resistant to the nuclease degradation agent. Alternatively, a backbone of the affinity reagent consisting essentially of a L-deoxyribonucleic acid, a barcode oligonucleotide consisting essentially of a phosphorothioate-based nucleic acid, a label oligonucleotide consisting essentially of a D-deoxyribonucleic acid, and a nuclease such as DNase I as degradation agent. In this case, the D-DNA label oligonucleotide is sensitive to the degradation agent and is readily digested by the nuclease upon its addition. The remaining nucleic acids are resistant to the nuclease degradation agent. Further alternatively, a backbone of the affinity reagent consisting essentially of a D-deoxyribonucleic acid, a barcode oligonucleotide consisting essentially of a D-deoxyribonucleic acid, a label oligonucleotide consisting essentially of a phosphorothioate-based nucleic acid, and silver, iodine or mercury as the degradation agent. In this case, the phosphorothioate-based nucleic acid label oligonucleotide is sensitive to the degradation agent and is readily degraded by the degradation agent upon its addition. The remaining nucleic acids are resistant to the degradation agent. Yet further alternatively, a backbone of the affinity reagent consisting essentially of a L-deoxyribonucleic acid, a barcode oligonucleotide consisting essentially of a locked nucleic acid, a label oligonucleotide consisting essentially of a phosphorothioate-based nucleic acid, and silver, iodine or mercury as the degradation agent. In this case, the phosphorothioate-based nucleic acid label oligonucleotide is sensitive to the degradation agent and is readily degraded by the degradation agent upon its addition. The remaining nucleic acids are resistant to the degradation agent.

In a further aspect, a method for analysing a biological sample comprising at least a target analyte is provided. The method comprises: introducing into the biological sample at least a first marker, in particular components thereof. The first marker comprising an affinity reagent with a backbone specific to the target analyte and a first label with at least a first labelling moiety. In particular, by introducing the marker, the marker may bind to the respective target analyte in the biological sample. The method further includes generating a first readout of the biological sample with the at least one first marker. This step may include directing excitation light onto the biological sample to visualise an optically detectable label and generate a readout from light emitted by the label by means of a microscope, for example. The method further includes applying a degradation agent to the biological sample in order to remove the first label of the first marker. In particular, this may degrade and release the label oligonucleotide from the marker, in particular, from the affinity reagent of the marker. This step may optionally include washing the biological sample in order to remove the released labels. The method further includes introducing at least one second label with at least a second labelling moiety into the biological sample in order to generate a second marker, the second label comprising a second label oligonucleotide configured to hybridise to the barcode oligonucleotide of the affinity reagent (or to a barcode oligonucleotide of an affinity reagent), and generating a second readout of the biological sample with the second marker. This enables robustly analysing the biological sample, in particular the target analytes. The use of the marker with sensitive and resistant oligonucleotides enables easily degrading specific elements of the marker in order to enable cycling of labels that are associated with the affinity reagent. Preferably, the label oligonucleotides are sensitive to the degradation agent.

The second label oligonucleotide may be identical to the label oligonucleotide of the first label. Thus, the first and second label oligonucleotides may bind to the same barcode oligonucleotide. Alternatively, the affinity reagent may comprise several barcode oligonucleotides that differ in their nucleotide sequence. In this case, the first label oligonucleotide and the second oligonucleotide may have a complementary sequence to a specific one of the several barcode oligonucleotides.

It is particularly preferred that the readout generated is an optical readout, for example, generated by means of a microscope.

Generally, a plurality of markers may be introduced into the biological sample, the markers being specific to respective target analytes, in order to identify a large number of target analytes at the same time.

Preferably, the target analyte is identified and/or localised within the biological sample based on the first label and the second label, in particular the labelling moieties, associated with the target analyte in the first readout and second readout. This enables identifying and/or localising the target analyte with improved precision. Since the affinity reagent is bound to the target analyte, the first and the second label introduced into the biological sample are associated with that target analyte via the affinity reagent.

Preferably, in the step of introducing the at least one first marker, the affinity reagent of the at least one first marker is introduced prior, in particularly separately, to the first label of the first marker, and the affinity reagent is immobilised in the biological sample prior to introducing the first label with the first labelling moiety into the biological sample. This enables introducing the marker into the biological sample separately as smaller individual elements that may penetrate the biological sample easier and then generating the marker in situ. The affinity reagent may be immobilised by crosslinking it with the biological sample, or parts thereof. Preferably, the affinity reagent is immobilised after it has bound to the target analyte. This keeps the affinity reagent in place, which may be particularly beneficial in case a large number of rounds of cyclical labelling is performed subsequently. The label is preferably introduced after crosslinking the affinity reagent.

Preferably, the first labelling moiety and the second labelling moiety have different detectable properties. This enables easily distinguishing between the labelling moieties. For example, the labelling moieties may be fluorophores with different, distinguishable emission wavelengths, excitation wavelengths, or emission duration such as e.g. fluorescence lifetime.

Preferably, after the step of generating the second readout, the steps of applying the degradation agent to degrade the second label oligonucleotide, adding at least one further label with a further labelling moiety, and generating a further readout of the biological sample, are iteratively repeated at least once. This enables robustly analysing the biological sample, in particular the target analytes. In particular, this enables identifying and/or localising the target analyte with improved precision. Particularly in case a biological sample with a large number of target analytes is analysed, each target analyte may be marked with a specific marker and in order to identify and/or localise each target analyte with improved precision. By iteratively removing and attaching different labels to the respective affinity reagents, the target analytes may be identified and/or located with improved precision. The further label preferably has different detectable properties than first and second label, in particular their respective labelling moieties, in order to distinguish the labels.

In a further aspect, a kit is provided comprising at least one marker and a degradation agent. Alternatively, the kit may comprise an affinity agent, a label, and a degradation agent. The label comprising a label oligonucleotide and at least one label moiety. The label is further configured to constitute a marker when the label is connected with the affinity reagent.

### Short Description of the Figures

Hereinafter, specific embodiments are described referring to the drawings, wherein:
- Figure 1: is a schematic view of an affinity reagent and a label of a marker, and
- Figure 2: is a schematic view of steps of a method for analysing a biological sample with the marker.

### Detailed Description

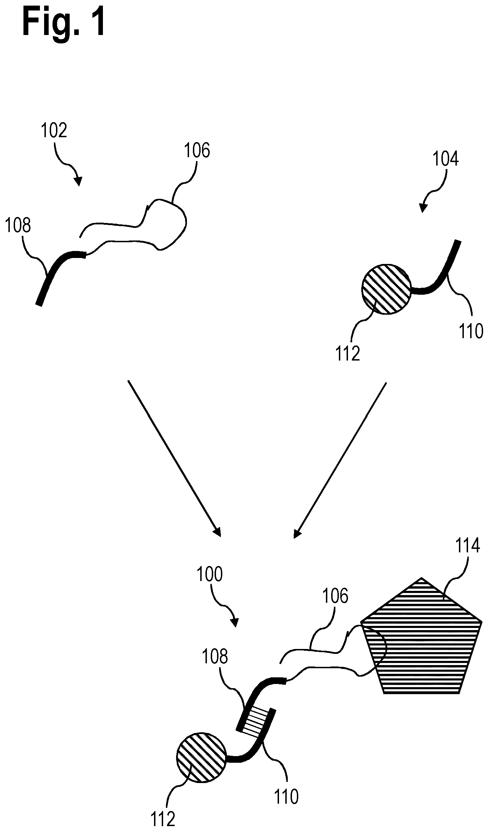

Figure 1 is a schematic view of an affinity reagent 102 and a label 104 of a marker 100. The affinity reagent 102 comprises a backbone 106 and a barcode oligonucleotide 108. The backbone 106 consists essentially of a nucleic acid analogue. Similarly, the barcode oligonucleotide 108 consists essentially of a nucleic acid analogue.

Generally, nucleic acid analogues are compounds which are structurally similar to naturally occurring RNA and DNA. Nucleic acids are chains of nucleotides, which are composed of three parts: a phosphate backbone, a pentose sugar, either ribose or deoxyribose, and one of four nucleobases. An analogue may have any of these altered. The nucleic acid analogue may be an artificial nucleic acid or a xeno nucleic acid. Specific examples of nucleic acid analogues include: Nucleic acids with modified sugars, such as L-DNA comprising L-pentose sugars, or modification of the ribose at the 2'position with for example 2'-amino, 2'-O-methyl, 2'-fluoro modifications. Further, nucleic acids with modified phosphate backbone such as phoshporothioate (PT) or boranophosphate (BP) modifications. These nucleic acid analogues are generally resistant to naturally occurring nucleases. This provides a particularly robust affinity reagent that is particularly suited for applications, such as staining, in biological samples, which regularly comprise naturally occurring nucleases.

The backbone 106 of the affinity reagent 102 may be a nucleic acid analogue based aptamer, for example. The barcode oligonucleotide 108 may be covalently attached to the backbone 106 of the affinity reagent 102. The barcode oligonucleotide 108 may be attached to any of a phosphate backbone, a pentose sugar, or a nucleobase, or their respective analogues, of the nucleic acid analogue of the backbone 106 of the affinity reagent 102. For example, attachment of the barcode oligonucleotide 108 may be by an azide/alkyne click chemistry. Alternatively, attachment of the barcode oligonucleotide 108 may be by a near-covalent interaction between streptavidin-biotin. To that end, the barcode oligonucleotide 108 may be covalently attached to one of (monovalent) streptavidin and biotin and the backbone 106 of the affinity reagent 102 may be covalently attached to the other one of (monovalent) streptavidin and biotin. When assembling the affinity reagent 102, the streptavidin binds to the biotin resulting in the attachment of the barcode oligonucleotide 108 to the backbone 106. This may be particularly advantageous, when a large library of aptamers needs to connected to a barcode each.

The barcode oligonucleotide 108 may be attached to the backbone 106 during solid-phase synthesis of the backbone.

The barcode oligonucleotide 108 may be attached to the backbone 106 after synthesis of the backbone using for example a click chemistry like azide/alkyne-modifications to connect barcode oligonucleotide 108 and backbone 106 covalently.

The barcode oligonucleotide 108 may be attached to the backbone 106 via an affinity interaction, for example the backbone 106 may be biotinylated and the barcode oligonucleotide may be conjugated to a (monovalent) Streptavidin.

The barcode oligonucleotide 108 may be attached to the backbone 106 using a linking oligonucleotide configured to hybridize with a portion of the barcode oligonucleotide and a portion of the backbone forming a triplex structure wherein, barcode oligonucleotide 108 and backbone 106 are directly opposed. In this case the open "nick", i.e. the missing covalent link in the backbones of the barcode oligonucleotide 108 and backbone 106 can be closed using T4 DNA ligase or a similar DNA ligase. When large libraries have to be connected to respective barcodes suitable universal adapter sequences on the barcode oligonucleotide 108 and backbone 106 may be leveraged to this end.

The backbone 106 of the affinity reagent 102 is configured to specifically bind to a particular target analyte 114, such as a protein of a biological sample. In particular, the backbone 106 may form or fold into a complex structure or conformation. The complex structure of the backbone 106 of the affinity reagent 102 may be a secondary, tertiary and/or quaternary structure of the backbone 106 nucleic acid analogue. Thus, the backbone 106 of the affinity reagent 102 has a secondary, tertiary or quaternary structure, in particular. The affinity reagent 102 binds specifically to the target analyte 114 due to the complex structure of the backbone 106 of the affinity reagent 102. Specifically, individual, discontinuous nucleotides of the nucleic acid analogue of the backbone 106 bind to the target analyte 114. This binding may comprise intermolecular forces such as hydrogen bonding, dipole-dipole interactions, and van der Waals forces. This is in contrast to a nucleic acid analogue hybridising, which is characterised by a continuous sequence of nucleotides of the nucleic acid analogue hybridising to a target analyte, in particular a complementary nucleotide sequence of the target analyte.

The binding of the backbone 106 of the affinity reagent 102 to the target analyte 114 based on the complex structure of the backbone 106 enables particular high affinity binding and specificity to the target analyte 114.

The nucleic acid analogue of the backbone 106 may comprise multiple individual sequences of a nucleic acid analogue. These individual sequences may bind to each other and form a quaternary structure.

The label 104 comprises a label oligonucleotide 110 and a labelling moiety 112. The label oligonucleotide 110 and the barcode oligonucleotide 108 are complementary to each other such that they may hybridise to each other. Preferably, the barcode oligonucleotide 108 sequence is specific to the backbone 106 of the affinity reagent 102. Thus, the identity of the target analyte 114 that the backbone 106 of the affinity reagent 102 is specific to may be encoded in the barcode oligonucleotide 108. Similarly, detectable properties of the labelling moiety 112 may be encoded in the barcode oligonucleotide 108. As the consequence, these properties or identities may similarly be encoded in the complementary label oligonucleotide 110.

The labelling moiety 112 is detectable by a particular detection instrument. For example, the labelling moiety 112 may be a fluorophore detectable by a fluorescent microscope.

The marker 100 is generated by attaching the label 104 to the affinity reagent 102 based on the complementary barcode oligonucleotide 108 and label oligonucleotide 110.

One of the label oligonucleotide 110 and the barcode oligonucleotide 108 is sensitive to a degradation agent whereas the other one of the label oligonucleotide 110 and the barcode oligonucleotide 108 is resistant to the degradation agent. The backbone 106 of the affinity reagent 102 is preferably resistant to the degradation agent. This enables efficiently removing the label 104 from the affinity reagent 102 by adding the degradation agent. For example, in case the label oligonucleotide 110 is sensitive to the degradation agent and the barcode oligonucleotide 108 is resistant to the degradation agent, the label oligonucleotide 110 is degraded by the degradation agent resulting in the removal or the dissociation of the label 104 from the affinity reagent 102. To this end, the label oligonucleotide 110 preferably consists essentially of a natural nucleic acid such as D-DNA.

In a particular preferred embodiment, the backbone 106 consists essentially of a L-DNA and the barcode oligonucleotide 108 consists essentially of a PT-modified or BP-modified nucleic acid analogue. In this case the label oligonucleotide 110 may be a D-DNA and the degradation agent may be a naturally occurring nuclease, such as DNase I. The use of a L-DNA and a PT- or BT-modified nucleic acid analogue for the backbone 106 and the barcode oligonucleotide 108, respectively, has the advantages that (A) both are highly resistant to nucleases and will be stable in a cyclic staining process and (B) that the modified barcode oligonucleotide 108 comprising a D-DNA will not hybridize with an aptamer backbone comprising L-DNA.

In an alternative embodiment, the label oligonucleotide 110 may be a PT-modified nucleic acid analogue sensitive to an iodine degradation agent. The backbone 106 and the barcode oligonucleotide 108 may be an iodine resistant nucleic acid analogue such as L-DNA.

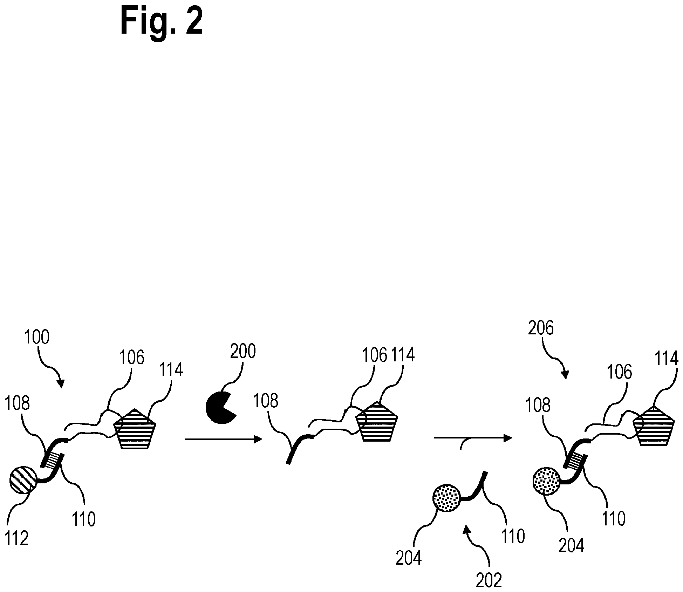

Figure 2 is a schematic view of steps of a method for analysing a biological sample (not shown in the Figures) with the marker 100. The marker 100 is initially introduced into the biological sample and binds to the target analyte 114. Similarly, a plurality of (different) markers may be introduced into the biological sample with the plurality of markers having markers that are specific to different target analytes. The labels of the plurality of markers may differ according to their specificity to a particular target analyte. A readout, such as an optical readout, may then be generated of the biological sample. For example, in case the labelling moiety 112 is a fluorophore, the readout may be an optical readout generated by means of a fluorescent microscope. In the readout the detectable label may be identified.

In a next step, a degradation agent 200 may be applied to the biological sample. For example, the degradation agent 200 may a nuclease such as DNase I. The label oligonucleotide 110 is sensitive to the degradation agent 200, for example, due to the label oligonucleotide 110 being a D-DNA. The resistant barcode oligonucleotide 108 and the backbone 106 of the affinity reagent 102 may be a L-DNA resistant to the nuclease degradation agent 200. The digestion of the label oligonucleotide 110 by the nuclease degradation agent 200 results in the removal of the label 104 from the affinity reagent 102 of the marker 100. Optionally, the elements 110, 112 of the label 104 remaining in the biological sample may be washed out.

In a next step a second label 202 is introduced into the biological sample comprising the label oligonucleotide 110 and a second labelling moiety 204. The label oligonucleotide 110 readily hybridises to the barcode oligonucleotide 108 of the affinity reagent 102. This generates a second marker 206 in the biological sample attached to the target analyte 114. Preferably, the second labelling moiety 204 has different detectable properties in order to distinguish the first label 102 initially attached to the target analyte 114 via the affinity reagent 102 and the second label 202. In case of fluorophore labelling moieties, the detectable properties may include an excitation wavelength, an emission wavelength and an emission duration. A second readout may be generated of the biological sample with the second marker 206 attached to the target analyte 114. Based on the first and the second readout, the target analyte 114 may be identified and/or localised in the biological sample.

The method may be repeated by removing the second label 202 with the degradation agent 200 and iteratively adding and removing at least one further label to the biological sample. This enables reliably identifying and/or localising a large number of target analytes with a plurality of markers comprising different labels. In each iteration of the method, a label with particular detectable properties may be assigned to a particular affinity reagent and therefore target analyte. Based on this assignment, the target analyte may be identified and/or localised in the generated readouts.

Identical or similarly acting elements are designated with the same reference signs in all Figures. As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### Reference signs

- 100,206: Marker
- 102: Affinity reagent
- 104,202: Label
- 106: Backbone of affinity reagent
- 108: Barcode oligonucleotide
- 110: Label oligonucleotide
- 112, 204: Labelling moiety
- 114: Target analyte
- 200: Degradation agent

## Claims

1. An affinity reagent (102) for analysing a biological sample comprising:
a backbone (106), and
a barcode oligonucleotide (108) attached to the backbone (106),
wherein the backbone (106) consists essentially of a nucleic acid analogue, the backbone (106) is configured to specifically bind to the target analyte (114) by its complex structure, and
wherein the barcode oligonucleotide (108) consists essentially of a nucleic acid analogue.

2. The affinity reagent according to claim 1, wherein the backbone (106) comprises 10 to 100 nucleotides.

3. The affinity reagent according to one of the preceding claims, wherein the nucleic acid analogue of the backbone (106) comprises at least one of the following, a locked nucleic acid, a morpholino, a nucleic acid backbone comprising phosphorothioate modifications, a peptide nucleic acid, a bridged nucleic acid, a 2'O-methyl-stubstituted RNA, a glycol nucleic acid, a hexitol nucleic acid, an altritol nucleic acid, a threose nucleic acid, a cyclohexene nucleic acids, a fluoroarabinonucleic acid, a L-DNA.

4. The affinity reagent according to one of the preceding claims, wherein the nucleic acid analogue of the barcode oligonucleotide (108) comprises at least one of the following, a locked nucleic acid, a morpholino, a nucleic acid backbone comprising phosphorothioate modifications, a peptide nucleic acid, a bridged nucleic acid, a 2'O-methyl-stubstituted RNA, a glycol nucleic acid, a hexitol nucleic acid, an altritol nucleic acid, a threose nucleic acid, a cyclohexene nucleic acids, a fluoroarabinonucleic acid, a L-DNA.

5. The affinity reagent according to one of the preceding claims, wherein the backbone (106) is an aptamer.

6. The affinity reagent according to one of the preceding claims, wherein the barcode oligonucleotide (108) being attached to the backbone (106) is adapted to be connected to a label (104, 202) via a label oligonucleotide (110), the label (104, 202) comprising the label oligonucleotide (110) and at least one labelling moiety (112, 204), wherein the backbone (106) and the barcode oligonucleotide (108) are resistant to a degradation agent (200) and the label oligonucleotide (110) is sensitive to the degradation agent (200).

7. A marker (100, 206) for analysing a biological sample comprising:
an affinity reagent (102) according to one of the preceding claims, and
a label (104, 202) comprising a label oligonucleotide (110) and at least one labelling moiety (112),
wherein the label oligonucleotide (110) and the barcode oligonucleotide (108) of the affinity reagent (102) are complementary to each other, and the label (104, 202) is hybridised to the affinity reagent (102) based on complementary base pairing between the label oligonucleotide (110) and the barcode oligonucleotide (108), and
wherein one of the label oligonucleotide (110) and the barcode oligonucleotide (108) is sensitive to a degradation agent (200) and the other one of the label oligonucleotide (110) and the barcode oligonucleotide (108) is resistant to the degradation agent (200).

8. The marker according to claim 7, wherein the label oligonucleotide (110) is sensitive to the degradation agent (200) and the affinity reagent (102) is resistant to the degradation agent (200).

9. The marker according to one of the preceding claims 7 or 8, wherein the label oligonucleotide (110) consists essentially of a natural nucleic acid.

10. The marker according to one of the preceding claims 7 to 9, wherein the at least one labelling moiety (112, 204) is optically detectable.

11. The marker according to one of the preceding claims 7 to 10, wherein the label (104, 202) comprises a plurality of labelling moieties (112, 204).

12. The marker according to one of the preceding claims 7 to 11, wherein the degradation agent (200) is a nuclease.

13. A method for analysing a biological sample comprising at least a target analyte (114), the method comprising the steps:
introducing into the biological sample at least a first marker (100) according to one of the preceding claims 7 to 12 comprising an affinity reagent (102) with a backbone (106) specific to the target analyte (114) and a first label (104) with at least a first labelling moiety (112),
generating a first readout of the biological sample with the at least one first marker (100),
applying a degradation agent (200) to the biological sample in order to remove the first label (104) of the first marker (100),
introducing at least one second label (202) with at least a second labelling moiety (204) into the biological sample in order to generate a second marker (206), the second label (202) comprising a second label oligonucleotide (110) configured to hybridise to the barcode oligonucleotide (108) of the affinity reagent (102), and
generating a second readout of the biological sample with the second marker (206).

14. The method according to claim 13, wherein the target analyte (114) is identified based on the first label (104) and the second label (202) associated with the target analyte (114) in the first readout and second readout.

15. The method according to one of the preceding claims 13 or 14, wherein in the step of introducing the at least one first marker (100), the affinity reagent (102) of the at least one first marker (100) is introduced prior to the first label (104) of the first marker (100), and wherein the affinity reagent (102) is immobilised in the biological sample prior to introducing the first label (104) with the first labelling moiety (112) into the biological sample.

16. The method according to one of the preceding claims 13 to 15, wherein the first labelling moiety (112) and the second labelling moiety (204) have different detectable properties.

17. The method according to one of the preceding claims 13 to 16, wherein after the step of generating the second readout, the steps of applying the degradation agent (200) to degrade the second label oligonucleotide (110), adding at least one further label with a further labelling moiety, and generating a further readout of the biological sample, are iteratively repeated.

18. A kit comprising at least one marker (100, 206) according to one of the preceding claims 7 to 12 and a degradation agent (200), or
comprising an affinity agent (102) according to one of the preceding claims 1 to 6, a label (104, 202), and a degradation agent (200), wherein the label (104, 202) comprises a label oligonucleotide (110) and at least one label moiety (112, 204), and wherein the label (104, 202) is configured to constitute a marker (100, 206) according to one of the preceding claims 7 to 12 when the label (104, 202) is connected with the affinity reagent (102).
